# EUROPEAN PATENT SPECIFICATION

(11) **EP 3 597 156 B1**
(45) Date of publication and mention of the grant of the patent: **17.03.2021**
(21) Application number: 18183761.8
(22) Date of filing: 16.07.2018
(51) Int. Cl.: A61F 5/00

(54) **GASTROINTESTINAL IMPLANT AND METHOD FOR DEPLOYING THE SAME**
GASTROINTESTINALES IMPLANTAT UND VERFAHREN ZUR HERSTELLUNG DAVON
IMPLANT GASTRO-INTESTINAL ET SON PROCÉDÉ DE DÉPLOIEMENT

(43) Date of publication of application: 22.01.2020
(73) Proprietor: National Taiwan University of Science and Technology, Taipei 106 (TW)
(72) Inventor: TSAI, Hsieh-Chih, 235 New Taipei City (TW); CHANG, Hao-Ming, 114 Taipei City (TW); HUANG, Chun-Chiang, 235 New Taipei City (TW); ZHAN, Wei-Ping, 811 Kaohsiung City (TW); LEE, Ya-Lun, 807 Kaohsiung City (TW); CHAO, Hsiao-Ying, 238 New Taipei City (TW); TSAI, Chi-Yang, 414 Taichung City (TW)
(74) Representative: Zimmermann, Tankred Klaus

(56) References cited:
- WO-A1-2013/049779
- US-A1- 2005 055 039
- US-A1- 2009 299 486
- US-A1- 2013 079 603

## Description

### BACKGROUND

### Technical Field

The present invention relates to a gastrointestinal implant and methods for deploying the same within a patient's digestive tract. In particular, the present invention relates to a gastrointestinal implant for treatment of obesity and/or its comorbidities, such as diabetes.

### Related Art

Obesity is an overwhelmingly growing health concern which is estimated to be directly responsible for over $147B in healthcare costs or 3%-8% of the overall healthcare costs of certain countries. Because of the enormous strain associated with carrying this excess weight, organs are affected, as are the nervous and circulatory systems. In 2013, one of three deaths is directly related to obesity in US.

One of the principle costs to the healthcare system stems from the co-morbidities associated with obesity. Type-2 diabetes has climbed to 9.3% of the population. Of those persons with Type-2 diabetes, almost half are clinically obese, and two thirds are approaching obese. Other co-morbidities include hypertension, coronary artery disease, hypercholesteremia, sleep apnea and pulmonary hypertension.

Although the physiology and psychology of obesity are complex, the medical consensus is that the cause is quite simple-an over intake of calories combined with a reduction in energy expenditures seen in modern society. While the treatment seems quite intuitive, the institution of a cure is a complex issue that has so far vexed the best efforts of medical science.

The main treatment for obesity is to reduce body fat by ingesting fewer calories and increasing exercise. A beneficial side effect of exercise is to increase muscle, tendon, and ligament strength, which helps to prevent injury from accidents and vigorous activity. Diet and exercise programs produce an average weight loss of approximately 8% of total body mass (excluding results from those individuals who drop out of such programs). Not all dieters are satisfied with these results, but a loss of as little as 5% of body mass can create large health benefits. Much more difficult than reducing body fat is maintaining its absence. Eighty to ninety-five percent of those who lose 10% or more of their body mass through dieting regain their lost body mass within two to five years.

The body has systems that maintain its homeostasis, including body weight, at certain set points. Therefore, keeping weight off generally requires making exercise and healthy eating a permanent part of a person's lifestyle. Certain nutrients or chemicals, such as phenylalanine, are natural appetite suppressants which allow the body to reset its set point with regard to body weight. However, dieting, exercise, and/or appetite suppressants may not result in sufficient weight loss in patients with serious medical conditions.

An alternative solution is surgery. There have been many attempts in the past to surgically modify patients' anatomies to attack the consumption problem by reducing the desire to eat. Known bariatric surgery procedures include jejuno-ileal bypass, jejuno-colic shunt, biliopancreatic diversion, gastric bypass, Roux-en-Y gastric bypass, gastroplasty, gastric banding, vertical banded gastroplasty, and silastic ring gastroplasty. A more complete history of bariatric surgery can be found on the website of the American Society for Bariatric Surgery at http://www.asbs.org.

Although being able to achieve early weight loss, bariatric surgery most often cannot sustain weight reduction. The reasons are not all known, but are believed related to several factors. One of which is that the stomach stretches over time increasing volume while psychological drivers motivate patients to find creative approaches to literally eat around the smaller pouch.

There are currently two surgical procedures that successfully produce long-term weight loss; the Roux-en-Y gastric bypass and the biliopancreatic diversion with duodenal switch (BPD). Both procedures shorten the effective-length of intestine available for nutrient absorption. Bypassing the duodenum makes it more difficult to digest fats, high sugar and carbohydrate rich foods. In the BPD procedure, large lengths of jejunum are bypassed resulting in malabsorption and therefore, reduced caloric uptake. However, these procedures carry a heavy toll and often are reserved for the most morbidly obese as there are several serious side effects of prolonged malabsorption.

The morbidity rate for surgical procedures is alarmingly high with 11% requiring surgical intervention for correction. Early small bowel obstruction occurs at a rate of between 2-6% in these surgeries and mortality rates are reported to be approximately 0.5-1.5%. While surgery seems to be an effective answer, the current invasive procedures are not acceptable with these complication rates.

A number of endoscopic therapies have been proposed for the treatment of obesity which, unlike invasive therapies, are minimally invasive and are often reversible. These therapies as currently in use, however, are significantly limited in their efficacy as well as safety. For instant, the failure rate for intestinal barrier sleeves is relatively high, primarily due to patient intolerance and gastrointestinal bleeding particularly at the anchorage points in the intestines.

US 2013/079603 A1 describes an intragastric anchor implant device with an elongate member having proximal and distal ends with atraumatic features disposed thereon. The atraumatic features inhibit damage to the tissues of the stomach and the gastrointestinal tract and help anchor the device. The elongate member may be substantially rigid to resist bending and engage a tissue of the stomach or gastrointestinal tract to prevent passage therethrough and may include one or more bends to conform to the shape of the tract. The anchor implant device may be coupled with a therapeutic device, such as an intestinal bypass sleeve with a sliding seal, such as an expandable structure, that secures the sleeve to direct a flow of food particles from the stomach therethrough, for use in treating various disorders, including obesity and diabetes. US 2013/079603 A1 shows the preamble of indepedent claim 1.

US 2009/299486 A1 describes a gastrointestinal prosthesis for restraining the rate of the gastric digestion which consists of a compressible proximal member connected by a string to a distal member, to be introduced into the intestine. The proximal member has an internal space, which is opened to the gastric lumen and may include passageways for the gastric content as well as open grooves or niches disposed on its external wall. The volume of a proximal member compressed by the gastric wall cannot get smaller than a lower threshold. Anchoring the gastrointestinal prosthesis at its desired location is accomplished by means of the proximal member the geometrical shape of which conforms the geometrical shape of a portion of the gastric lumen; as well as by means of the geometrical shape of the distal member which conforms the geometrical shape of a segment of the duodenum and/or a segment of the intestine.

US 2013/049779 A1 describes an integrated circuit comprising a first pair of switching devices arranged in series between positive and negative supply terminals is disclosed. The integrated circuit is switchable between an operational mode, in which the first pair of switching devices are driven to couple either the positive or negative supply terminal to an output terminal, and a test mode, in which a current source on the integrated circuit is driven to cause a desired current to flow in a first one of the first pair of switching devices.

US 2005/055039 A1 describes a device for performing one or more functions in a gastrointestinal tract of a patient includes an anchoring member and at least one actuator, sensor, or combination of both coupled with the anchoring device. The anchoring device is adapted to maintain at least part of the device within a pyloric portion of the patient's stomach and to intermittently engage, without directly attaching to, stomach tissue. Actuators perform any suitable function, such as transmitting energy to tissue, acting as a sleeve to reduce nutrient absorption, occupying space in the stomach, eluting a drug and/or the like. Sensors may be adapted to sense any suitable patient characteristic within the patient's gastrointestinal tract, such as pH, temperature, bile content, nutrient content, fats, sugars, alcohol, opiates, drugs, analytes, electrolytes and/or hemoglobin.

### SUMMARY

It is an object of the present invention to provide a perorally deployable device for the treatment of obesity and/or its comorbidities, as well as a way to mitigate the risk of position a bypass tube within the digestive tract of a patient.

This object is achieved by a gastrointestinal implant according to claim 1.

The present invention provides a gastrointestinal implant comprises a transpyloric anchor and a sleeve. The transpyloric anchor comprises a central stem, a major ring, a plurality of major branches, a minor ring and a plurality of minor branches. The central stem has a proximal end and a distal end. The major ring is movably disposed on and surrounds to the central stem. The plurality of major branches extend from the proximal end of the central stem and connect with the major ring. The minor ring is disposed at the distal end of the central stem. The plurality of minor branches extend from the distal end of the central stem end and connect with the minor ring. The sleeve has a head portion and a tail portion, and the sleeve is coupled to the minor ring of the transpyloric anchor. While no force applied to the major branches, the proximal end of the central stem, the major branches and the major ring form a hollow sphere.

In one embodiment, the gastrointestinal implant is biodegradable and materials of the gastrointestinal implant comprise biodegradable polyester.

In one embodiment, the materials of the gastrointestinal implant further comprise at least 2% barium sulfate.

In one embodiment, the materials of the gastrointestinal implant further comprise surfactant.

In one embodiment, while a force applied to the major branches, the proximal end of the central stem approaches major ring to form a plurality of chyme orifices, and the amount of the major branches and the chyme orifices is identical.

In one embodiment, while no force applied to the major branches, a length of each major branch is longer than a distance between the proximal end and the major ring.

In one embodiment, a distance of each major branch is identical to the adjacent major branches.

In one embodiment, an amount of the plurality of major branches is 3 to 6.

In one embodiment, an amount of the plurality of major branches is 6.

In one embodiment, the sleeve is configured to has a thickness of at least 0.0001 mm but no more than 3 mm.

In one embodiment, the sleeve is configured to has length of at least 40 cm but no more than 150 cm.

In one embodiment, the sleeve has a tensile strength of at least 1MPa but no more than 1 GPa.

In one embodiment, the head portion of the sleeve is thicker than rest of the sleeve.

In one embodiment, the inner surface of the sleeve has one or more frictional patterns.

In one embodiment, the outer surface of the sleeve has one or more frictional patterns.

In one embodiment, the head portion of the sleeve is integrated with the minor ring of the transpyloric anchor

In one embodiment, the head portion of the sleeve is adhesively attached to the minor ring of the transpyloric anchor.

In one embodiment, the head portion of the sleeve is mechanically attached to the minor ring of the transpyloric anchor.

In one embodiment, the gastrointestinal implant further comprises a deployment device. The deployment device guides the gastrointestinal implant to a position near pylorus. While the deployment device covers a portion of the plurality of major branches, a covering space of the transpyloric anchor decreased, and the plurality of major branches are parallel to the central stem.

In one embodiment, the transpyloric anchor is made using three-dimensional printing technology.

The foregoing objectives and summary provide only a brief introduction to the present invention. To fully appreciate these and other objects of the present invention as well as the invention itself, all of which will become apparent to those skilled in the art, the following detailed description of the invention and the claims should be read in conjunction with the accompanying drawings. Throughout the specification and drawings identical reference numerals refer to identical or similar parts.

Many other advantages and features of the present invention will become apparent to those versed in the art upon making reference to the detailed description and the accompanying sheets of drawings in which a preferred structural embodiment incorporating the principles of the present invention is shown by way of illustrative example.

### BRIEF DESCRIPTION OF THE DRAWINGS

The embodiments will become more fully understood from the detailed description and accompanying drawings, which are given for illustration only, and thus are not limitative of the present invention, and wherein:
FIG. 1 is a schematic diagram illustrating a conventional transpyloric anchor.
FIG. 2A is a schematic diagram illustrating a gastrointestinal implant according to an exemplary embodiment the present invention.
FIG. 2B is a schematic diagram illustrating a gastrointestinal implant deployed at the pylorus according to an exemplary embodiment the present invention.
FIG. 3A is a sectioned view illustrating a coupling between the sleeve and the transpyloric anchor according to an embodiment of the present invention.
FIG. 3B to 3D are schematic diagrams illustrating frictional patterns at the head portion of the sleeve according to embodiments of the present invention.
FIG. 4A-4C are schematic diagrams illustrating different amounts of the major branches according to an embodiment of the present invention.
FIG. 5A is a schematic diagram illustrating a deployment device for the gastrointestinal implant according to an embodiment of the present invention.
FIG. 5B is a schematic diagram illustrating a deployment device covering a part of the gastrointestinal implant according to an embodiment of the present invention.
FIG. 5C to 5F are schematic diagrams illustrating the deployment of the gastrointestinal implant using the deployment device according to an embodiment of the present invention.
FIG. 6A to 6B are schematic diagrams illustrating the deployment of the gastrointestinal implant without sleeve using the deployment device according to an embodiment of the present invention.
FIGS. 7 and 8 are flowcharts illustrating deployment methods for the gastrointestinal implant.

### DETAILED DESCRIPTION OF THE INVENTION

The various embodiments will be described in detail with reference to the accompanying drawings. Wherever possible, the same reference numbers will be used throughout the drawings to refer to the same or like parts. References made to particular examples and implementations are for illustrative purposes, and are not intended to limit the scope of the invention or the claims.

The word "exemplary" is used herein to mean "serving as an example, instance, or illustration." Any implementation described herein as "exemplary" is not necessarily to be construed as preferred or advantageous over other implementations.

The word "substantially" is used herein to mean "of considerable value, or of real importance." Any implementation described herein as "substantially" is not necessarily to be construed as preferred or advantageous over other implementations.

The word "mechanically" is used herein to mean "by employing mechanical means, or achieved in a mechanical way." Any implementation described herein as "mechanically" is not necessarily to be construed as preferred or advantageous over other implementations.

The word "fluid" is used herein to mean "gas, liquid, or gel." Any implementation described herein as "fluid" is not necessarily to be construed as preferred or advantageous over other implementations.

One concept of the present invention is to set up a barrier between the duodenum (the most digestive organ of human body) and the food that a patient eats. Another concept of the present invention is to set up another barrier between the orifice of the pylorus and the food that a patient eats (to reduce the area of the orifice of the pylorus). In this way, those barriers will block the duodenum from digesting the chyme and reduce the rate of the food entering the pylorus, and thus preventing the patient from taking in excess calories, which will help the patient to manage his/her blood sugar level or body weight. Such barrier is often deployed via operational procedures in order to position the barrier at the right place, and retrieved once the patient finishes his/her treatment session. The present invention adapts biodegradable material into such barrier to ensure that the barrier will hydrolyzed in the patient's body by itself without the need of retrieval. The present invention also adapts radio-opaque material into the barrier to monitor degradation progression in order to detect barrier displacement or unwanted side effect, such as ileus.

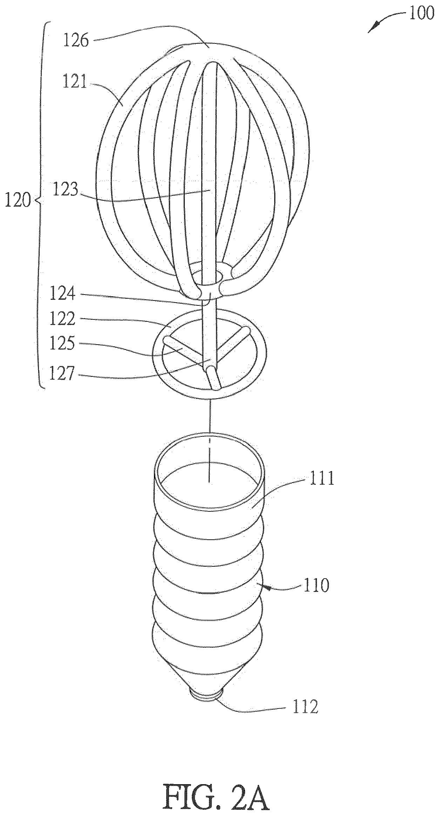

Please refer to FIG. 2A, which illustrates a schematic diagram of a gastrointestinal implant 100 according to an exemplary embodiment the present invention. The gastrointestinal implant 100 comprises a transpyloric anchor 120 and a sleeve 110. The sleeve 110 is configured to be positioned in a patient duodenum in order to serve as a barrier for blocking absorption. More specifically, the sleeve 110 has a head portion 111 at its proximal end, and a thickness of the head portion 111 is identical or thicker than the rest of the sleeve 110 and is coupled to the transpyloric anchor 120, such as coupled to the minor ring 122 of the transpyloric anchor 120. The sleeve 110 also has a tail portion 112 at its distal end. The transpyloric anchor 120 comprises a plurality of major branches 121, a minor ring 122, a central stem 123, a major ring 124, a plurality of minor branches 125. In a preferable embodiment, the plurality of major branches 121, the major ring 124 and central stem 123 can block a part of the foods from entering the pylorus 400. The plurality of minor branches 125 are configured to support structure of the minor ring 122.

In a preferable embodiment, the transpyloric anchor 120 is made using three-dimensional printing technology.

The central stem 123 has a proximal end 126 and a distal end 127. The major ring 124 is movably disposed on and surrounds to the central stem 123. By the configuration of the major ring 122 surrounding to (and not contacting) the central stem 123, the major ring 122 can therefore restrictly move or slide between the proximal end 126 and the distal end 127 of the central stem 123. The plurality of major branches 121 may be flexible and may extend, or preferably integrally extend from the proximal end 126 of the central stem 123 and connect with the major ring 124. More specifically, if the distance between the proximal end 126 and the major ring 122 is shorter than the length of the major branches 121, the major branches 121 bends, and if the distance between the proximal end 126 and the major ring 122 is equal to the length of the major branches 121, the major branches 121 straighten. The minor ring 122 is disposed at the distal end 124 of the central stem 123.

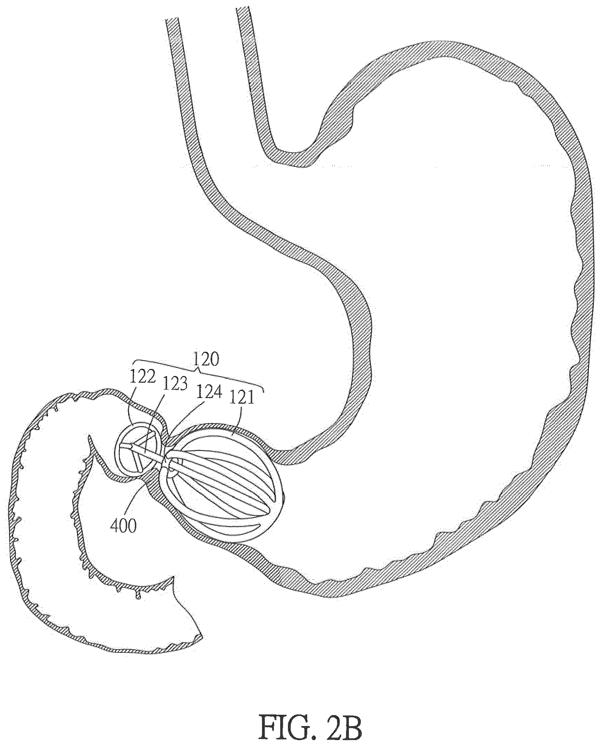

Please refer to FIG. 2B, which illustrates a schematic diagram of a gastrointestinal implant 100 deployed at the pylorus 400 according to an exemplary embodiment the present invention. The transpyloric anchor 120 is used to keep the sleeve 110 (not shown) in place at the duodenum, otherwise the sleeve 110 (not shown) might be displaced due to bowel movement. The plurality of major branches 121 is configured to be stuck at the orifice of the pylorus 400 at gastro side and the minor ring 122 is configured to be stuck at the orifice of the pylorus 400 at the intestinal side. By the configuration of the major ring 124 and the plurality of major branches 121, the foods (such as chymes) are only allow to pass the space between the plurality of major branches 121 and the area between the major ring 124 and the central stem 123, and therefore reduce the rate of the food entering the pylorus 400. When no force (such as a pressure generated by the accumulation of chymes) applied to the major branches 121, the proximal end 126 of the central stem 123, the major branches 121 and the major ring 124 form a hollow sphere having a covering area. For example, the covering area of the hollow sphere can bigger than the space of the orifice of the pylorus 400 to prevent major branches from entering the pylorus 400.

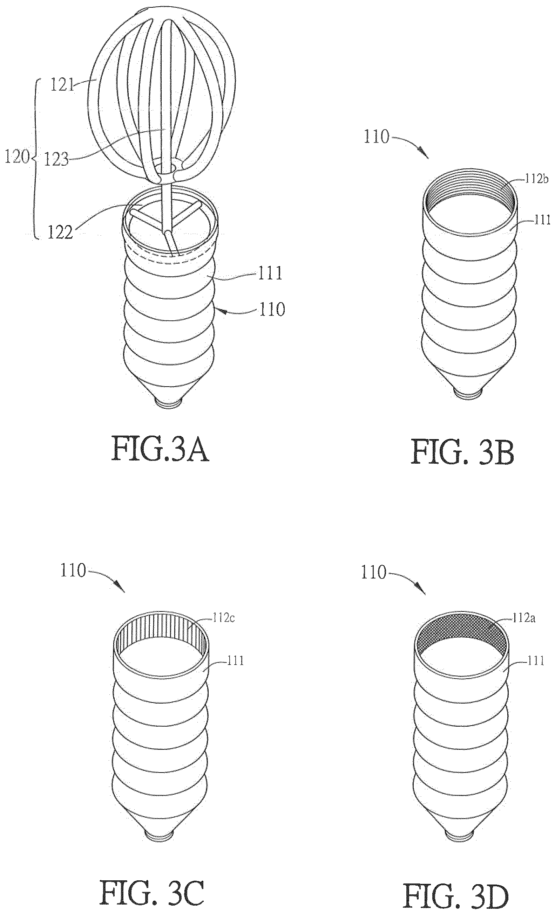

Please refer to FIG. 3A, which illustrates a sectioned view of a coupling between the sleeve 110 and the transpyloric anchor 120 according to an embodiment of the present invention. In an embodiments, the head portion 111 of the sleeve 110 is integrated with the minor ring 122 of the transpyloric anchor 120. For example, sleeve 110 and the minor ring 122 could be integrally formed in one piece. In another embodiments, the head portion 111 of the sleeve 110 is adhesively attached to the minor ring 122 of the transpyloric anchor 120 by biocompatible adhesives Ad, especially for binding the minor ring 122 and the sleeve 110. For the purpose of the present invention, the biocompatible adhesive Ad should be non-toxic to human body. However, the type of the adhesives used should not be a limitation of the present invention. In another embodiments, the head portion 111 of the sleeve 110 is mechanically attached to the minor ring 122 of the transpyloric anchor 120 by additional fixing element. However, the type of the additional fixing element used should not be a limitation of the present invention. The materials of the fixing elements can comprise plastic, metal or biodegradable materials. In this way, the sleeve 110 will be stuck with the minor ring 122. If the sleeve 110 were to slip off the transpyloric anchor 120, the head portion 111 would provide higher tensile strength which in turn prevents the sleeve 110 from slipping out of the transpyloric anchor 120.

In other embodiments, the inner surface of the sleeve 110 at the proximal end may have one or more frictional patterns. Please refer to FIG. 3B to 3D, which are schematic diagrams illustrating frictional patterns at the head portion 111 of the sleeve 110 according to embodiments of the present invention. The frictional patterns 112a, 112b, 112c will provide more frictional forces at the interface of the between the head portion 111 and the minor ring 122, if the sleeve 110 were to slip off the transpyloric anchor 120. The frictional patterns 112a, 112b, 112c including annular, linear, and grid types may be formed by molding, injection molding or engraving.

Please note that, the above-mentions patterns are for illustrative purpose only, and not meant to be a limitation of the present invention. For example, the frictional patterns 112a, 112b, 112c may also be adapted on the outer surface of the sleeve 110 at the proximal end to increase the friction force at the interface between the sleeve 110 and the duodenal wall, directly.

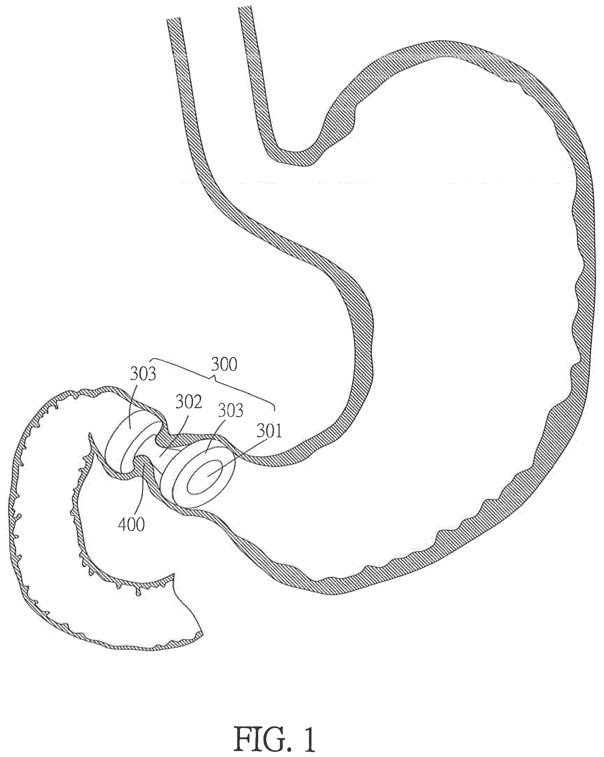

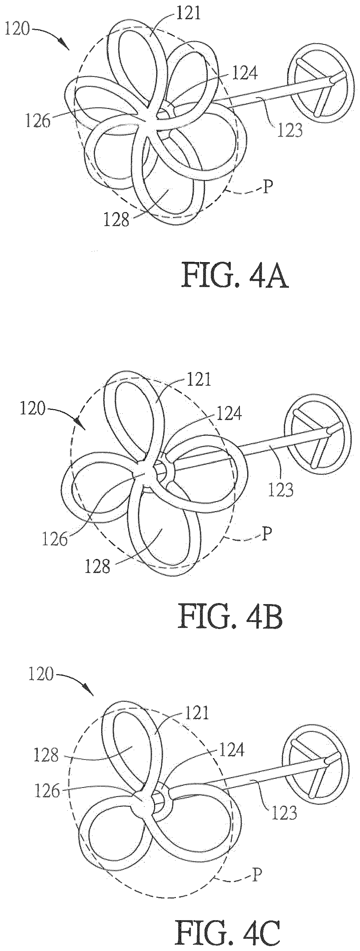

Please refer to FIG. 4A-4C, which are schematic diagrams illustrating different amounts of the major branches 121 according to embodiments of the present invention. The amounts of major branches 121 of the transpyloric anchor 120 is not limited. Specifically, amounts of major branches 121 of the transpyloric anchor 120 may be 3 to 6, preferably 4, 5 or 6, more preferably 6. Please note that, FIG. 4A-4C also illustrating the configuration of major branches 121 being pressed by a force, e.g. a pressure generated by the accumulation of chymes. For example, when a pressure generated by the accumulation of chymes applied to the major branches 121, the proximal end 126 of the central stem 123 will gradually approaches and even contacts with major ring 124 to form a plurality of chyme orifices 128, and an amount of the major branches 121 and the chyme orifices 128 is identical. The major branches 121 which forms the chyme orifices 128 also works like a barrier between the pylorus and the food that a patient eats, and can provide the effect of reducing the rate of the entering of chyme to the duodenum. When no force or pressure generated by the accumulation of chyme applied to the major branches, the major branches revert to its original configuration, e.g. the hollow sphere shape shown in FIG.2A. Please note that, the plurality of major branches 121 may form a virtual circular plane P. The more major branches 121 or chyme orifices 128 in the virtual circular plane P, the more hindrance for chymes to enter the pylorus 400. More specifically, due to volume of the major branches 121 (which blocks the chymes), the more amounts of the major branches 121 can make chyme more difficult to enter the pylorus 400 or enter the pylorus 400 slower. Please note that, the conventional transpyloric anchor 300 is provided with two inflatable rings 301 connected by the connecting means 302 (please referring to FIG. 1). Although the connecting means forms a lumen 302 to allow the chymes to enter the pylorus 400, the gradually increased pressure generated by the chymes may apply to the inflatable ring surface outside the pylorus 400. In the end, the inflatable ring 301 may be pushed into the pylorus 400, causing unwanted result for the treatment. To solve the disadvantages of the conventional transpyloric anchor 300, by the configuration of movable major ring 124 and hollow sphere provided by the proximal end 126 of the central stem 123, the major branches 121 and the major ring 124, it is advantageous that, (1) the major branches 121 can make different degrees of bending against the pressure caused by the accumulation of the chymes so as to prevent the transpyloric anchor 120 from being pushed into the pylorus 400; (2) the major branches 121 can block the chymes from entering the pylorus 400, but the hollow area (such as the space between each major branch 121) can allow the chymes to enter the pylorus 400.

The sleeve 110 and the transpyloric anchor 120 are made from biodegradable material, such as biodegradable polyester (e.g., polycaprolactone (PCL)). Due to its biocompatibility, mechanical strength, and hydrophobicity on the surface, biodegradable polyester is highly suitable for making implant that could last for years, while at the same time causes minimum risk to the patient since it will eventually be hydrolyzed in the body. The biodegradability of the sleeve 110 means that the patient won't suffer a second procedure to retrieve the sleeve 110 after his/her session finished. However, unexpected incidents may still occur during the sessions, which requires surgical procedure to replace, reposition, or take out the sleeve 110 ahead of time. For example, if the rate of degradation of the sleeve 110 is faster than planned or the patient need to extend his/her session, the physician may need to replace a new sleeve with the old one. In another example, if the sleeve 110 is twisted or displaced in the duodenum due to the bowel movement or the failure of the transpyloric anchor 120, the patient may need an emergency procedure to fix the sleeve 110.

It is clear that there is a need for monitoring the sleeve 110 after implantation. It is therefore, the material used to make the sleeve 110 should be not only biodegradable but also radio-opaque. In a preferable embodiment, the formulation of the sleeve 110 comprises at least 2% radio-opaque material, such as barium sulfate, and some surfactant. However, the type of the radio-opaque material and surfactant used should not be a limitation of the present invention. In this way, the rate of the degradation of the sleeve 110 can be monitored by periodically scanning the patient's abdomen with X-rays. The opaqueness of the sleeve 110 showed on the X-ray images can be used to determine the degree of the degradation. After the treatment session, if the sleeve 110 is hydrolyzed as expected, there is no need to retrieve the sleeve 110. The patient should only be examined periodically to make sure the degradation process is on schedule until the sleeve 110 is 100% degraded or excreted.

In addition, in order to facilitate the deployment while maintaining the functionality of the sleeve 110, the configuration of the sleeve 110 should be constrained. For example, the thickness of the sleeve 110 should be 0.0001 mm to 3 mm, 0.001 mm to 3 mm, 0.01 mm to 3 mm, 0.01 mm to 2 mm, 0.01 mm to 1 mm, such that the sleeve 110 is durable enough but the tensile strength of the sleeve 110 will not be too large to effect normal bowel movement.

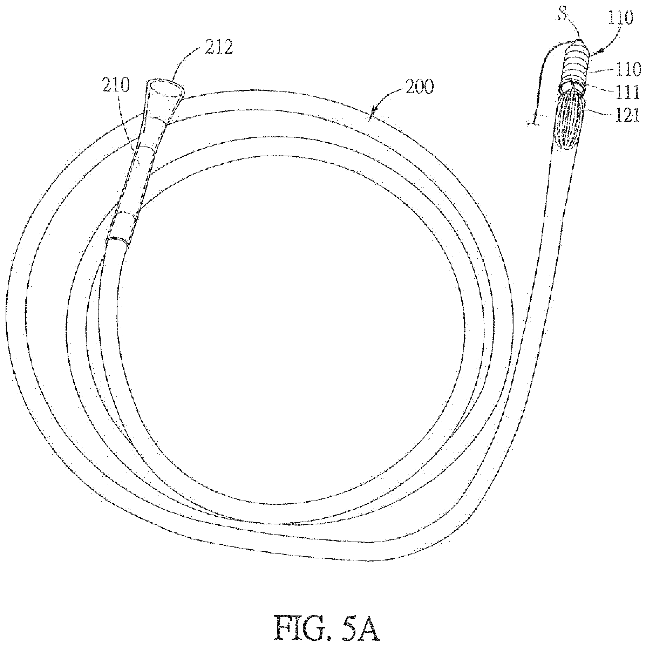

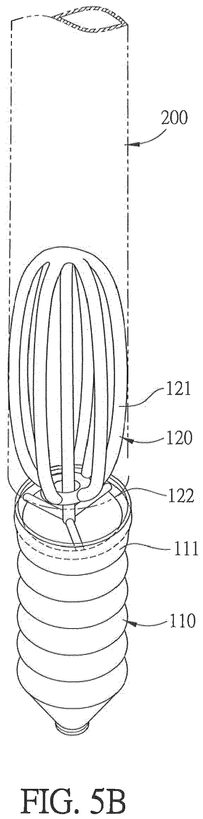

The preferable tensile strength of the sleeve 110 is between 1MPa to 1GPa according to the present invention. The length of the sleeve 110 should be at least 40cm but no more than 150cm which is long enough to shield the most part of the duodenum but not too long to make it challenging to deploy the sleeve 110. The diameter of the sleeve 110 should be at least 1 cm when fully inflated. Please refer to FIG. 5A to FIG. 5F. FIG. 5A is a schematic diagram illustrating a deployment device 200 for the gastrointestinal implant 100 according to an embodiment of the present invention. FIG. 5B to 5E illustrate the deployment of the gastrointestinal implant 100 using the deployment device according to an embodiment of the present invention. In FIG. 5A, the deployment device 200 may comprises a delivering catheter 210 and a fluid inlet 212. In a preferable embodiment, the deployment device 200 is a flexible tube which allow fluid to pass through. The length of the deployment device 200 is preferably. The diameter of the deployment device 200 is suitable for covering the plurality of major branches 121.

In FIG. 5B, at the start of the deployment, the tail portion 112 of the sleeve 110 is sealed until the gastrointestinal implant 100 is guided to a position near pylorus 400. In a preferable embodiment, the tail portion 112 of the sleeve 110 is temporarily sealed by tying a slipknot S (as shown in FIG. 5A) at the tail portion 112 of the sleeve 110 using a thread. In this way, the tail portion 112 of the sleeve 110 can be unsealed simply by pulling the slipknot S (as shown in FIG. 5A). When the deployment device 200 covers a portion of the plurality of major branches 121, a covering space of the transpyloric anchor 120 decreased, and the plurality of major branches 121 are approximately parallel to the central stem 123. In this way, it is easier for the physician to maneuver the deployment device 200. In the other embodiment, the tail portion 112 of the sleeve 110 can also be sealed by biodegradable resin.

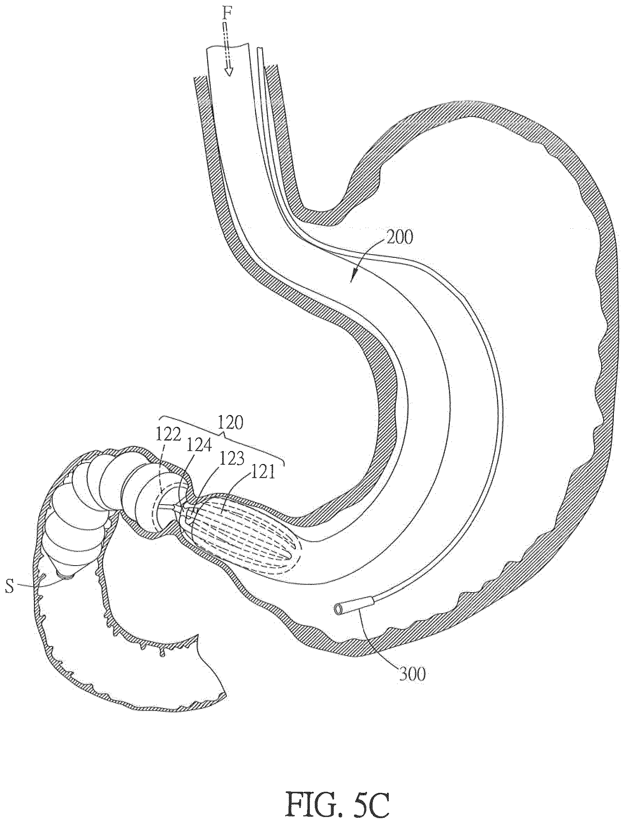

In FIG. 5C, with the help of an endoscope 300, the gastrointestinal implant 100 is delivered perorallly down the GI tract and passing through the pylorus 400 by carefully pushing the delivering catheter 210 until the minor ring 122 passes through the orifice of the pylorus 400.

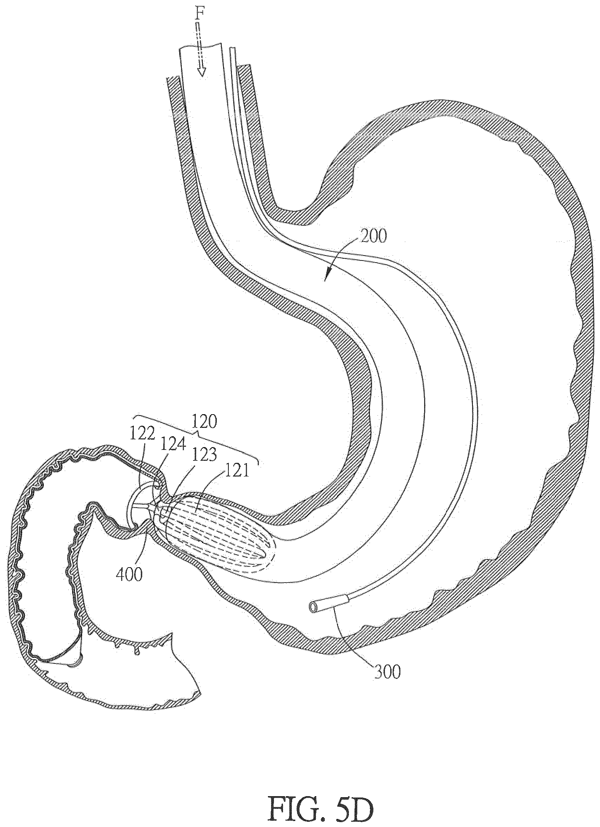

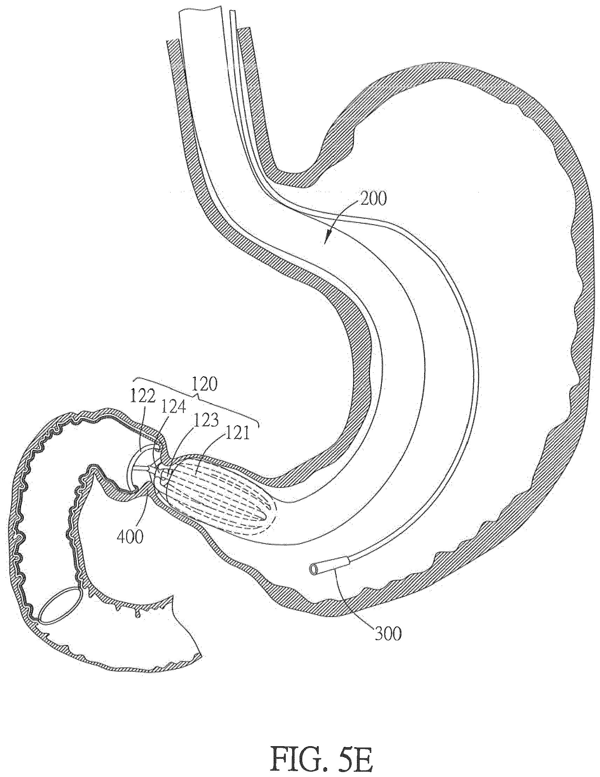

In FIG. 5D, the delivering catheter 210 starts receiving fluid F from the first fluid inlet 212, and thus the received fluid F is fed into the sleeve 110. As the fluid F filling the sleeve 110, the fluid F pressure accumulates and thus pushes the folded sleeve 110 to elongate down toward the jejunal. In FIG. 5E, once the sleeve 110 is fully extended, the temporary seal at the tail portion 112 of the sleeve 110 is unsealed and discharges the fluid F from the sleeve 110 to the GI.

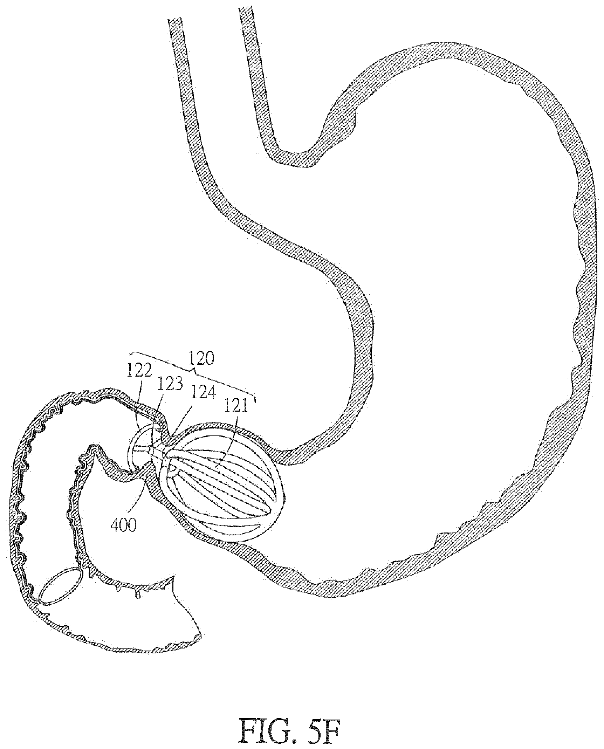

In FIG. 5F, after removing the deploying device 200, the deployment of the gastrointestinal implant 100 is completed. Please note that, the deploying device 200 may be removed by pulling the deploying device 200 out of the GI tract by force, and thereby detaching the deploying device 200 off the transpyloric anchor 120.

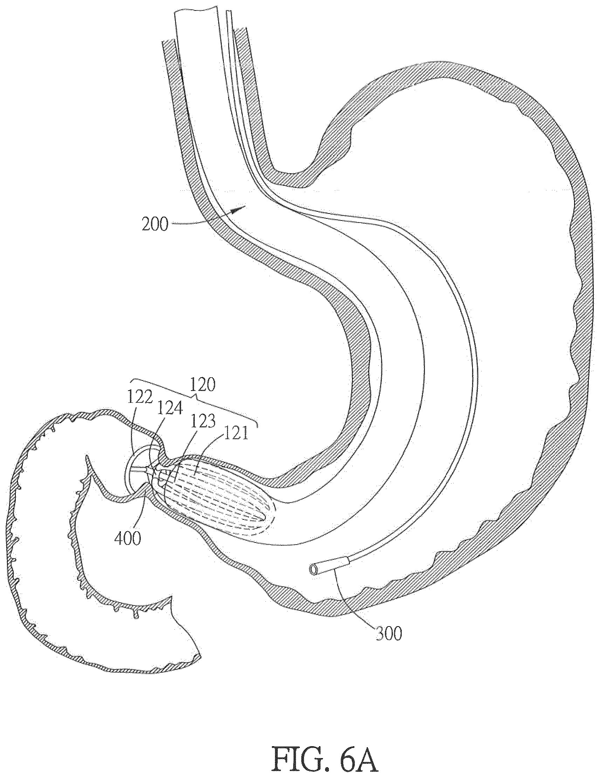

FIG. 6A to 6B illustrate another deployment of the gastrointestinal implant 100 without sleeve 110 using the deployment device according to an embodiment of the present invention.

In FIG. 6A, with the help of an endoscope 300, the gastrointestinal implant 100 without sleeve 110 is delivered perorallly down the GI tract and passing through the pylorus 400 by carefully pushing the delivering catheter 210 until the minor ring 122 passes through the orifice of the pylorus 400.

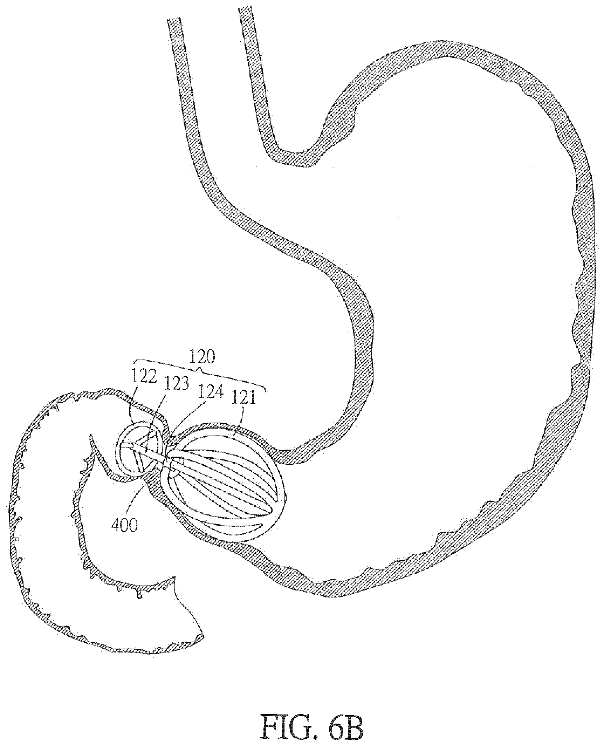

In FIG. 6B, after the gastrointestinal implant 100 without sleeve 110 is well-positioned, the deploying device 200 is removed, and the deployment of the gastrointestinal implant 100 is completed.

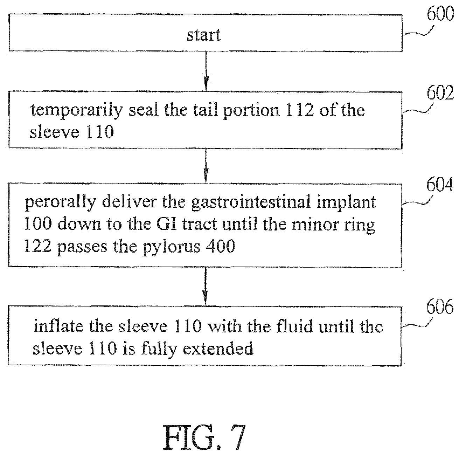

Please refer to FIG. 7, which is a flowchart illustrating a deployment method for the gastrointestinal implant 100. Provided that the result is substantially the same, the steps are not required to be executed in the exact order shown in FIG. 7. The exemplary method may be employed by the gastrointestinal implant 100 shown in FIG. 2, and may be briefly summarized as below.

Step 600: start.

Step 602: temporarily seal the tail portion 112 of the sleeve 110.

Step 604: perorally deliver the gastrointestinal implant 100 down to the GI tract until the minor ring 122 passes the pylorus 400.

Step 606: inflate the sleeve 110 with the fluid F until the sleeve 110 is fully extended.

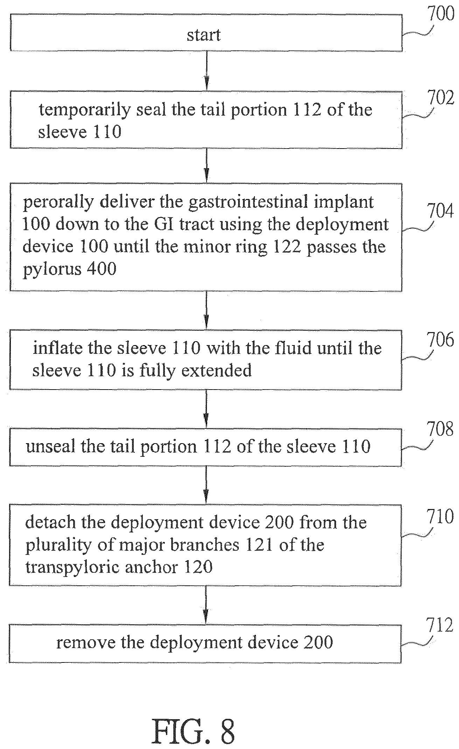

More specifically, in a case that the deployment device 200 is employed in the process, please refer to FIG. 8, which is a flowchart illustrating a deployment method for the gastrointestinal implant 100 using deployment device 200. Provided that the result is substantially the same, the steps are not required to be executed in the exact order shown in FIG. 8. The exemplary method may be briefly summarized as below.

Step 700: start.

Step 702: temporarily seal the tail portion 112 of the sleeve 110.

Step 704: perorally deliver the gastrointestinal implant 100 down to the GI tract using the deployment device 200 until the minor ring 122 passes the pylorus 400.

Step 706: inflate the sleeve 110 with the fluid F until the sleeve 110 is fully extended.

Step 708: unseal the tail portion 112 of the sleeve 110.

Step 710: detach the deployment device 200 from the plurality of major branches 121 of the transpyloric anchor 120.

Step 712: remove the deployment device 200.

In a preferable embodiment, the step of temporarily sealing the tail portion 112 of the sleeve 110 comprises step of tying a slipknot at the tail portion 112 of the sleeve 110 using a thread.

In a preferable embodiment, the method of deploying the gastrointestinal implant further comprises step of unsealing the tail portion of the sleeve 110 by untying the slipknot.

In a preferable embodiment, the thread used to tie the slipknot may be biodegradable.

Those who have ordinary skilled in the art should readily understood the process summarized by the FIG.7 and FIG.8 after reading the above paragraph. Detailed description is omitted here for brevity.

Although the invention has been described with reference to specific embodiments, this description is not meant to be construed in a limiting sense. Those skilled in the art will readily observe that numerous modifications and alterations of the device and method may be made while retaining the teachings of the invention. Accordingly, the above disclosure should be construed as limited only by the metes and bounds of the appended claims.

## Claims

1. A gastrointestinal implant (100), comprising:
a transpyloric anchor (120), which is comprising:
a central stem (123) having a proximal end (126) and a distal end (127);
a major ring (124) which is movably disposed on and surrounding to the central stem (123);
a plurality of major branches (121) extending from the proximal end (126) of the central stem (123) and connecting with the major ring (124);
a minor ring (122) which is disposed at the distal end (127) of the central stem (123); and
a plurality of minor branches (125) extending from the distal end (127) of the central stem (123) and connecting with the minor ring (122); and
a sleeve (110) having a head portion (111) and a tail portion (112), and the sleeve (110) is coupled to the minor ring (122) of the transpyloric anchor (120),
**characterized in that**
while no force applied to the major branches (121), the proximal end (126) of the central stem (123), the major branches (121) and the major ring (124) form a hollow sphere.

2. The gastrointestinal implant (100) of claim 1, wherein the gastrointestinal implant (100) is biodegradable and materials of the gastrointestinal implant (100) comprise biodegradable polyester.

3. The gastrointestinal implant (100) of claim 1, wherein the proximal end (126) of the central stem (123) is configured to contact the major ring (124) to form a plurality of chyme orifices (128), and an amount of the major branches (121) and the chyme orifices (128) is identical.

4. The gastrointestinal implant (100) of claim 1, wherein while no force applied to the major branches (121), a length of each major branch is longer than a distance between the proximal end (126) and the major ring (124).

5. The gastrointestinal implant (100) of claim 1, wherein a distance of each major branch (121) is identical to the adjacent major branches (121).

6. The gastrointestinal implant (100) of claim 1, wherein an amount of the plurality of major branches (121) is 6.

7. The gastrointestinal implant (100) of claim 1, wherein the sleeve (110) is configured to has a thickness of at least 0.0001 mm but no more than 3 mm.

8. The gastrointestinal implant (100) of claim 1, wherein a head portion (111) of the sleeve (110) is integrated with the minor ring (122) of the transpyloric anchor (120).

9. The gastrointestinal implant (100) of claim 1, wherein a head portion (111) of the sleeve (110) is mechanically attached to the minor ring (122) of the transpyloric anchor (120).

10. The gastrointestinal implant (100) of claim 1, wherein the gastrointestinal implant (100) further comprising:
a deployment device (200) for guiding the gastrointestinal implant (100) to a position near pylorus,
wherein, while the deployment device (200) covers a portion of the plurality of major branches (121), a covering space of the transpyloric anchor (120) decreased, and the plurality of major branches (121) are parallel to the central stem (123).

## Patentansprüche

1. Ein gastrointestinales Implantat (100), das folgende Merkmale aufweist:
einen transpylorischen Anker (120), der folgende Merkmale aufweist:
einen zentralen Stamm (123) mit einem proximalen Ende (126) und einem distalen Ende (127);
einen Hauptring (124), der beweglich auf dem zentralen Stamm (123) angeordnet ist und denselben umgibt;
eine Mehrzahl von Hauptzweigen (121), die sich von dem proximalen Ende (126) des zentralen Stamms (123) erstrecken und eine Verbindung mit dem Hauptring (124) eingehen;
einen Nebenring (122), der an dem distalen Ende (127) des zentralen Stamms (123) angeordnet ist; und
eine Mehrzahl von Nebenzweigen (125), die sich von dem distalen Ende (127) des zentralen Stamms (123) erstrecken und eine Verbindung mit dem Nebenring (122) eingehen; und
eine Hülse (110) mit einem Kopfabschnitt (111) und einem Endabschnitt (112), und wobei die Hülse (110) mit dem Nebenring (122) des transpylorischen Ankers (120) gekoppelt ist,
**dadurch gekennzeichnet, dass**
wenn keine Kraft auf die Hauptzweige (121) ausgeübt wird, das proximale Ende (126) des zentralen Stamms (123), die Hauptzweigen (121) und der Hauptring (124) eine hohle Kugel bilden.

2. Das gastrointestinale Implantat (100) gemäß Anspruch 1, wobei das gastrointestinale Implantat (100) biologisch abbaubar ist und Materialien des gastrointestinalen Implantats (100) biologisch abbaubares Polyester aufweisen.

3. Das gastrointestinale Implantat (100) gemäß Anspruch 1, wobei das proximale Ende (126) des zentralen Stamms (123) dazu konfiguriert ist, den Hauptring (124) zu berühren, um eine Mehrzahl von Chymus-Öffnungen (128) zu bilden, und eine Menge der Hauptzweige (121) und der Chymus-Öffnungen (128) identisch ist.

4. Das gastrointestinale Implantat (100) gemäß Anspruch 1, wobei, wenn keine Kraft auf die Hauptzweige (121) ausgeübt wird, eine Länge jedes Hauptrings größer als ein Abstand zwischen dem proximalen Ende (126) und dem Hauptring (124) ist.

5. Das gastrointestinale Implantat (100) gemäß Anspruch 1, wobei ein Abstand jedes Hauptrings (121) zu den benachbarten Hauptzweigen (121) identisch ist.

6. Das gastrointestinale Implantat (100) gemäß Anspruch 1, wobei eine Menge der Mehrzahl von Hauptzweigen (121) 6 beträgt.

7. Das gastrointestinale Implantat (100) gemäß Anspruch 1, wobei die Hülse (110) dazu konfiguriert ist, eine Dicke von zumindest 0,0001 mm, jedoch nicht mehr als 3 mm aufzuweisen.

8. Das gastrointestinale Implantat (100) gemäß Anspruch 1, wobei ein Kopfabschnitt (111) der Hülse (110) mit dem Nebenring (122) des transpylorischen Ankers (120) integriert ist.

9. Das gastrointestinale Implantat (100) gemäß Anspruch 1, wobei ein Kopfabschnitt (111) der Hülse (110) mechanisch an dem Nebenring (122) des transpylorischen Ankers (120) angebracht ist.

10. Das gastrointestinale Implantat (100) gemäß Anspruch 1, wobei das gastrointestinale Implantat (100) ferner folgendes Merkmal aufweist:
eine Bereitstellungsvorrichtung (200) zum Führen des gastrointestinalen Implantats (100) zu einer Position in der Nähe des Pylorus,
wobei, wenn die Bereitstellungsvorrichtung (200) einen Abschnitt der Mehrzahl von Hauptzweigen (121) bedeckt, ein Abdeckraum des transpylorischen Ankers (120) abnimmt und die Mehrzahl von Hauptzweigen (121) parallel zu dem zentralen Stamm (123) ist.

## Revendications

1. Implant gastro-intestinal (100), comprenant:
une ancre transpylorique (120), qui comprend:
une tige centrale (123) présentant une extrémité proximale (126) et une extrémité distale (127);
une bague principale (124) qui est disposée de manière déplaçable sur et entourant la tige centrale (123);
une pluralité de branches principales (121) s'étendant à partir de l'extrémité proximale (126) de la tige centrale (123) et se connectant à la bague principale (124);
une bague mineure (122) qui est disposée à l'extrémité distale (127) de la tige centrale (123); et
une pluralité de branches mineures (125) s'étendant à partir de l'extrémité distale (127) de la tige centrale (123) et se connectant à la bague mineure (122); et
un manchon (110) présentant une partie de tête (111) et une partie de queue (112), et le manchon (110) est couplé à la bague mineure (122) de l'ancre transpylorique (120),
**caractérisé par le fait que**
tandis qu'il n'est pas appliqué de force sur les branches principales (121), l'extrémité proximale (126) de la tige centrale (123), les branches principales (121) et la bague principale (124) forment une sphère creuse.

2. Implant gastro-intestinal (100) selon la revendication 1, dans lequel l'implant gastro-intestinal (100) est biodégradable et les matériaux de l'implant gastro-intestinal (100) comprennent du polyester biodégradable.

3. Implant gastro-intestinal (100) selon la revendication 1, dans lequel l'extrémité proximale (126) de la tige centrale (123) est configurée pour entrer en contact avec la bague principale (124) pour former une pluralité d'orifices de chyme (128), et une quantité des branches principales (121) et des orifices de chyme (128) est identique.

4. Implant gastro-intestinal (100) selon la revendication 1, dans lequel, tandis qu'il n'est pas appliqué de force sur les branches principales (121), une longueur de chaque branche principale est plus longue qu'une distance entre l'extrémité proximale (126) et la bague principale (124).

5. Implant gastro-intestinal (100) selon la revendication 1, dans lequel une distance de chaque branche principale (121) est identique aux branches principales adjacentes (121).

6. Implant gastro-intestinal (100) selon la revendication 1, dans lequel une quantité de la pluralité de branches principales (121) est de 6.

7. Implant gastro-intestinal (100) selon la revendication 1, dans lequel le manchon (110) est configuré de manière à présenter une épaisseur d'au moins 0,0001 mm, mais de pas plus de 3 mm.

8. Implant gastro-intestinal (100) selon la revendication 1, dans lequel une partie de tête (111) du manchon (110) est intégrée à la bague mineure (122) de l'ancre transpylorique (120).

9. Implant gastro-intestinal (100) selon la revendication 1, dans lequel une partie de tête (111) du manchon (110) est fixée mécaniquement à la bague mineure (122) de l'ancre transpylorique (120).

10. Implant gastro-intestinal (100) selon la revendication 1, dans lequel l'implant gastro-intestinal (100) comprend par ailleurs:
un dispositif de déploiement (200) destiné à guider l'implant gastro-intestinal (100) vers une position proche du pylore,
dans lequel, tandis que le dispositif de déploiement (200) recouvre une partie de la pluralité de branches principales (121), un espace de recouvrement de l'ancre transpylorique (120) est diminué, et la pluralité de branches principales (121) sont parallèles à la tige centrale (123).
